# EUROPEAN PATENT SPECIFICATION

(11) **EP 2 057 953 B1**
(45) Date of publication and mention of the grant of the patent: **06.05.2020**
(21) Application number: 07806371.6
(22) Date of filing: 30.08.2007
(51) Int. Cl.: A61B 18/14, A61B 1/00

(54) **TREATMENT TOOL FOR ENDOSCOPE**
BEHANDLUNGSWERKZEUG FÜR EIN ENDOSKOP
OUTIL DE TRAITEMENT POUR ENDOSCOPE

(30) Priority: 30.08.2006 JP 2006234159
(43) Date of publication of application: 13.05.2009
(73) Proprietor: Jichi Medical University, Shimotsuke-shi, Tochigi 329-0498 (JP)
(72) Inventor: YAMAMOTO, Hironori, Shimotsuke-shi Tochigi 329-0498 (JP)
(74) Representative: Klunker IP Patentanwälte PartG mbB
(86) International application number: PCT/JP2007/066894
(87) International publication number: WO 2008/026689

(56) References cited:
- EP-A1- 1 013 228
- WO-A1-2005/079901
- JP-A- 2000 139 942
- JP-A- 2003 290 248
- JP-A- 2004 248 911
- US-A- 4 532 924
- US-A- 6 071 277
- US-A1- 2004 167 514

## Description

### Technical Field

The present invention relates to a treatment instrument for endoscope, more particularly, to a treatment instrument for endoscope which is used in endoscopic submucosal dissection (ESD).

### Background Art

Endoscopic mucosal resection is known for its usefulness as a minimally invasive radical surgery that is a reliable treatment for neoplastic mucosal lesion such as early gastric cancer and early colorectal cancer. In recent years, a method called endoscopic submucosal dissection (ESD) has been developed and widely used as a reliable endoscopic demucosation method for en bloc resection of a wider lesion. The method includes an incision of mucous membrane around a tumor, and a cutting of a submucosal layer between the mucous membrane and a proper muscular layer for en bloc resection of tumor mucous membrane. The method enables a drawing of an incision line as intended, and ensures the extirpation of tumor, but at the same time, it involves tremendous technical difficulty, and also requires training and skill in the treatment using the method, and also there is another problem that the treatment takes a long time.

In order to solve the above problems, various treatment instruments for endoscope have been proposed. For example, the treatment instrument for endoscope described in Patent Document 1 is a hook knife formed with a bended bar having a high frequency electrode at the distal end thereof, and the treatment instrument is used by hooking the distal end of the hook knife over mucosal tissues, and drawing the mucosal tissues into a sheath of the knife to ablate the mucosal tissues. While, the treatment instrument for endoscope described in Patent Document 2 is an IT knife having a needle knife with an insulator at the distal end thereof, and the insulator prevents the treatment instrument from sticking into proper muscular layer. The treatment instruments for endoscope have been used to try to decrease any technical difficulty of endoscopic submucosal dissection.

Document US 6 071 277 A discloses a treatment instrument in which the treatment section comprises two ring electrodes which are held by a support structure having a bulb shape. US 4 532 924 A also discloses a treat instrument. This prior art instruments comprises a treatment section having an insulating body shaped similar to a bullet. On the surface of the insulating body there are provided plural electrodes either extending in the longitudinal direction of the bullet-shaped body or in the circumferential direction thereof. WO 2005/079901 A1 discloses a treatment instrument having a treatment section comprised of a cylindrical body carrying spiral electrodes.

The present invention was made in view of the above situation, and one object of the present invention is to provide a treatment instrument for endoscope that is suitable to endoscopic submucosal dissection.

### Means for Solving the Problems

The invention is as defined in the appended claims. A first aspect of the present invention is, in order to achieve the above object, provides a treatment instrument for endoscope having a conductor connected to a high-frequency source, on a treatment section provided at a distal end of an insertion section to be inserted into a body, in which the treatment section is configured in a sandwich structure comprising the conductor and non-conductors provided on the distal end side and a proximal end side of the conductor, and the conductor is exposed in a strip-shape at a side surface of the treatment section.

According to the first aspect of the present invention, the treatment section has a side surface that has a sandwich structure in which a strip-shaped conductor is sandwiched between non-conductors, thereby when the distal end of the treatment section is brought in contact with proper muscular layer, the non-conductor contacts the proper muscular layer, and the conductor does not. Therefore, any cutting of the proper muscular layer can be prevented without fail. Furthermore, according to the first aspect of the present invention, when the treatment section is laterally moved, a submucosal layer contacts the side surface of the treatment section, and the contacted part of the layer that comes in contact with the strip-shaped conductor is cut. Therefore, according to the first aspect of the present invention, without causing any damage on proper muscular layer, a submucosal layer can be cut.

In addition, in the first aspect of the present invention, the treatment section perpendicularly approaches and cuts a wall of digestive tract (mucous membrane or proper muscular layer), which enables an easy approach of the treatment section while the wall of digestive tract is squarely observed by the endoscope. Moreover, in the first aspect of the present invention, the treatment section is laterally moved for cutting, thereby a cutting can be achieved while the wall of digestive tract is squarely observed by the endoscope.

A second aspect of the present invention provides the treatment instrument for endoscope in the first aspect of the present invention, wherein the strip-shaped conductor is formed to extend around the side surface of the treatment section. According to the second aspect of the present invention, because the conductor is formed to extend around the side surfaces of the treatment section, the treatment section is provided with cutting ability at all of the side surfaces. Therefore, the treatment section moved in any lateral direction can cut a submucosal layer.

A third aspect of the present invention provides the treatment instrument for endoscope in the first or second aspect of the present invention, wherein the treatment section is formed to have a gear-like shape in which peaks and valleys are alternately arranged in a circumferential direction of the treatment section. According to the third aspect of the present invention, because the treatment section is formed to have a gear-like shape, a fibrous submucosal layer is likely to be hooked by the peaks of the treatment section when the treatment section is laterally moved, resulting in an effective cutting of the submucosal layer.

A fourth aspect of the present invention provides the treatment instrument for endoscope in one of the first to third aspect of the present invention, wherein the treatment section is supported via a swing mechanism for controlling a posture of the treatment section. According to the fourth aspect of the present invention, because the treatment section is supported via a swing mechanism, the postures of treatment section are under control, which facilitates the cutting process.

A fifth aspect of the present invention provides the treatment instrument for endoscope in one of the first to fourth aspect of the present invention, wherein the strip-shaped conductor includes at least three conductors that are disposed in parallel to each other and sandwich non-conductors therebetween, and the treatment instrument for endoscope includes a high-frequency current supply apparatus which selects two conductors including one at the most distal end side, out of the at least three conductors to apply a high frequency current for coagulation with using the selected two conductors as a pair of electrodes, and selects two conductors other than the one at the most distal end side, out of the at least three conductors to apply a high frequency current for cutting using the selected two conductors as a pair of electrodes.

According to the fifth aspect of the present invention, because the part for applying a high frequency current for coagulation is provided on more distal end side than the part for applying a high frequency current for cutting, the coagulated tissues are left on the distal end side (the proper muscular layer side) of the incision position, which considerably reduces a resulting amount of bleeding. In addition, according to the fifth aspect of the present invention, a coagulation position and an incision position are not coincident with each other, which facilitates the control of a depth of coagulation, and stops a resulting bleeding without fail, while preventing excess damage of tissues due to the coagulation. The fifth aspect of the present invention is a bipolar treatment instrument for endoscope.

A sixth aspect of the present invention provides the treatment instrument for endoscope in the fifth aspect of the present invention, wherein three conductors are provided as the conductor, and the high-frequency current supply apparatus selects a conductor on the most distal end side and a conductor on the most proximal end side to apply a high frequency current for coagulation, and selects a central conductor and the conductor on the most proximal end side to apply a high frequency current for cutting.

A seventh aspect of the present invention provides the treatment instrument for endoscope in the fifth aspect of the present invention, wherein four conductors are provided as the conductor, and the high-frequency current supply apparatus selects one or two pairs of conductors including a conductor on the most distal end side and a conductor on the most proximal end side to apply a high frequency current for coagulation, and selects two central conductors to apply a high frequency current for cutting. According to the seventh aspect of the present invention, the coagulated tissues are left also on the proximal end side (the mucous membrane side) of the incision position, which further reduces a resulting amount of bleeding. That is, according to the seventh aspect of the present invention, the part between the coagulated tissues on the distal end side and the coagulated tissues on the proximal end side is cut, which considerably reduces an amount of bleeding due to the cutting.

An eighth aspect of the present invention provides the treatment instrument for endoscope in one of the first to fourth aspect of the present invention, wherein the strip-shaped conductor includes at least two conductors that are disposed in parallel to each other and sandwich a non-conductor therebetween, and the treatment instrument for endoscope includes a high-frequency current supply apparatus which selects at least one conductor including a conductor on the most distal end side, out of the at least two conductors to apply a high frequency current for coagulation so that the selected conductor consists one of a pair of electrodes, and selects at least one conductor other than the conductor on the most distal end side, out of the at least two conductors to apply a high frequency current for cutting so that the selected conductor consists one of a pair of electrodes.

According to the eighth aspect of the present invention, because the part for applying a high frequency current for coagulation is provided on more distal end side than the part for applying a high frequency current for cutting, the coagulated tissues are left on the distal end side (the proper muscular layer side) of the incision position, which considerably reduces a resulting amount of bleeding. In addition, according to the eighth aspect of the present invention, a coagulation position and an incision position are not coincident with each other, which facilitates the control of a depth of coagulation, and stops the bleeding without fail, while preventing excess damage of tissues due to the coagulation. The eighth aspect of the present invention is a monoplar-type treatment instrument for endoscope.

A ninth aspect of the present invention provides the treatment instrument for endoscope in the eighth aspect of the present invention, wherein two conductors are provided as the conductor, and the high-frequency current supply apparatus selects the two conductors to apply a high frequency current for coagulation and selects a conductor on the proximal end side to apply a high frequency current for cutting.

A tenth aspect of the present invention provides the treatment instrument for endoscope in the eighth aspect of the present invention, wherein three conductors are provided as the conductor, and the high-frequency current supply apparatus selects a conductor on the most distal end side and a conductor on the most proximal end side to apply a high frequency current for coagulation, and selects a central conductor to apply a high frequency current for cutting. According to the tenth aspect of the present invention, the coagulated tissues are left also on the proximal end side (the mucous membrane side) of the incision position, which further reduces an amount of bleeding. That is, according to the tenth aspect of the present invention, the part between the coagulated tissues on the distal end side and the coagulated tissues on the proximal end side is cut, which considerably reduces an amount of bleeding due to the cutting.

An eleventh aspect of the present invention provides the treatment instrument for endoscope in one of the first to tenth aspect of the present invention, wherein the treatment section is coupled with a distal end portion of a wire which connects between the conductor and the high-frequency source, or with a bar-shaped electrode to which the wire is connected, and the wire is provided to be movable forward and backward relative to an insulative sheath into which the wire is inserted, and the distal end portion of the wire or the bar-shaped electrode can be adjusted to an exposed state or a non-exposed state. According to the eleventh aspect of the present invention, because an advancement of the wire relative to the sheath causes the wire or the bar-shaped electrode to be exposed, when a high frequency current flows through the wire, the conductor(s) on the side surface of the treatment section as well as the wire or the bar-shaped electrode are able to perform the cutting. Therefore, according to the present invention, the wire or bar-shaped electrode in an exposed state can be used for an incision of mucous membrane.

A twelfth aspect of the present invention provides the treatment instrument for endoscope in one of the one of the first to eleventh aspect of the present invention, wherein discontinuous projections are integrally formed with the non-conductor, on a proximal end surface of the treatment section. According to the twelfth aspect of the present invention, because the treatment section has a proximal end surface with a projection, the projection can be used for hooking and collecting submucosal layer, resulting in an effective operation for removing submucosal layer.

A thirteenth aspect of the present invention provides the treatment instrument for endoscope in one of the first to twelfth aspect of the present invention, wherein the conductor is exposed at a proximal end surface of the treatment section. According to the present invention, because the conductor is exposed at the proximal end surface of the treatment section, when a high frequency current is applied to the conductor, a cutting can be achieved by the proximal end surface of the treatment section as well as the side surface of the treatment section. Therefore, according to the present invention, a mucous membrane can be cut the conductor at the proximal end surface of the treatment section, concurrently with the cutting of a submucosal layer by the conductor at the side surface of the treatment section.

### Advantage of the Invention

According to the present invention, since a treatment section has a side surface that has a sandwich structure in which a strip-shaped conductor is sandwiched between non-conductors, a cutting of submucosal layer can be performed without causing any damage on proper muscular layer. Therefore, according to the present invention, endoscopic submucosal dissection can be performed in a quick and safe manner.

### Brief Description of the Drawings

Figure 1 is a perspective view showing a treatment instrument for endoscope of a first embodiment according to the present invention;
Figure 2 is a front view showing the treatment section of Figure 1 seen in the direction of the arrow A;
Figure 3 is a cross sectional view of the treatment section showing a cross section taken along the 3-3 line of Figure 2;
Figure 4 are views showing a method for operating a treatment instrument for endoscope of the present invention: Figure 4A shows a situation where the part around a lesion is marked; Figure 4B shows a situation where the lesion is bulged; Figure 4C shows a situation where an incision of mucous membrane is performed; Figure 4D shows a situation after the incision of mucous membrane; Figure 4E shows a situation where a submucosal layer is being cut; and Figure 4F shows a situation after the cutting of submucosal layer;
Figure 5 is a cross sectional view showing a state of cutting by a treatment section;
Figure 6 is a cross sectional view showing a bipolar-type treatment instrument for endoscope;
Figure 7 is a side view showing a treatment instrument for endoscope of a second embodiment according to the present invention;
Figure 8 is a cross sectional view showing the treatment section of Figure 7;
Figure 9 is a side view showing the treatment section of Figure 7 in another posture;
Figure 10 is a view of the treatment section of Figure 9 seen in the direction B;
Figure 11 is a plan view showing a treatment section having a swing mechanism which has a different configuration from that of Figure 10;
Figure 12 is a cross sectional view of the treatment section of Figure 10;
Figure 13 is a cross sectional view showing a treatment instrument for endoscope having a swing mechanism which has a different configuration from that of Figure 10;
Figure 14 is a cross sectional view showing a t treatment instrument for endoscope having a swing mechanism which has a different configuration from that of Figure 10;
Figure 15 is a side view showing a treatment section of a treatment instrument for endoscope of a third embodiment according to the present invention;
Figure 16 is a perspective view showing a treatment instrument for endoscope having a conductor at a sheath thereof;
Figure 17 is a cross sectional view showing a treatment instrument for endoscope having a function of high-frequency knife;
Figure 18 is a cross sectional view showing a treatment instrument for endoscope when a function of high-frequency knife being turned off, in Figure 17;
Figure 19 is a cross sectional view showing a treatment instrument for endoscope of a fourth embodiment according to the present invention;
Figure 20 is a cross sectional view showing the treatment section 20 of Figure 19;
Figure 21 is a perspective view showing a treatment section of a treatment instrument for endoscope of a fifth embodiment according to the present invention;
Figure 22 is a view of the treatment section of Figure 21 seen in the direction of the arrow B;
Figure 23 is a cross sectional view of a treatment section taken along the 23-23 line of Figure 22;
Figure 24 is a cross sectional view showing a situation where the treatment section of Figure 23 is advanced relative to a sheath;
Figure 25 is a cross sectional view of a treatment section showing a different projection from that of Figure 23;
Figure 26 is a cross sectional view of a treatment section showing a different projection from that of Figure 23;
Figure 27 is a cross sectional view of a treatment section which has a conductor of a different shape from that of Figure 23;
Figure 28 is a cross sectional view showing a treatment instrument for endoscope of a sixth embodiment according to the present invention; and
Figure 29 is a perspective view showing the treatment section of Figure 28.

### Description of Symbols

10 ... treatment instrument for endoscope, 12 ... insertion section, 14 ... hand-side operation section, 16 ... sheath, 18 ... wire, 20 ... treatment section, 20A ...peaks, 20B ...valleys, 28 ... non-conductor, 29 ... conductor, 30 ... conductor, 31 ... non-conductor, 32 ... non-conductor, 34 ... mucous membrane, 36 ... proper muscular layer, 38 ... submucosal layer, 46 ... first sleeve, 48 ... second sleeve, 90 ... treatment section, 91 to 94 ... conductor, 95 to 99 ... non-conductor, 120 ... treatment section, 120E ... projection, 122 ... non-conductor, 124 ... conductor, 124C ... proximal end side exposed surface, 126 ... non-conductor, 130 ... treatment section, 130E ... projection, 132 ... non-conductor, 134 ... conductor, 134A ... bar-shaped electrodes, 136 ... non-conductor, 138 ... connection pipe, 140 ... collar member

### Best Mode for Carrying Out the Invention

In accordance with the accompanying drawings, the preferred embodiments of a treatment instrument for endoscope according to the present invention will be described below in detail.

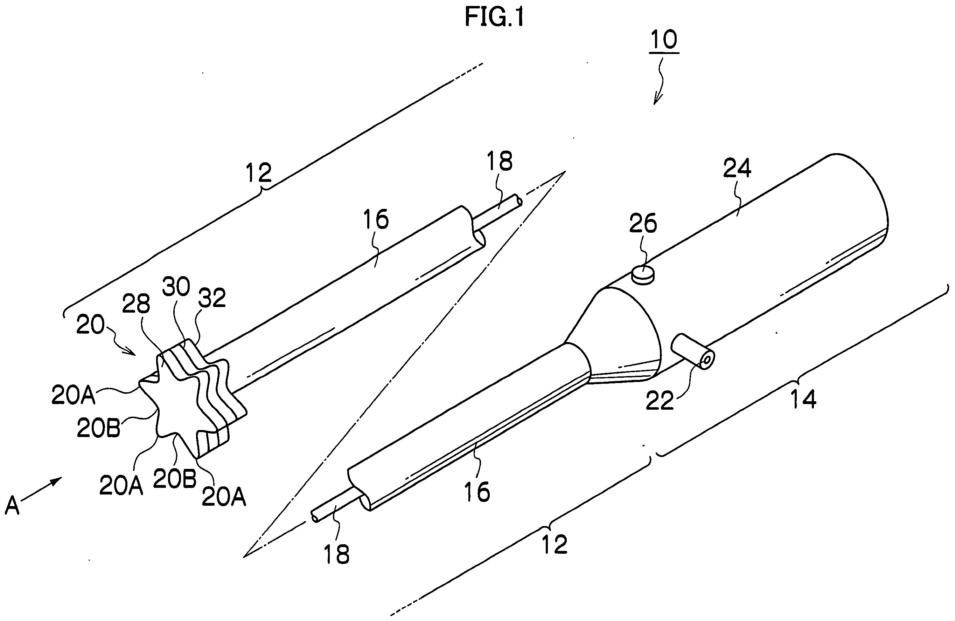

Figure 1 is a perspective view showing a first embodiment of a treatment instrument for endoscope 10. The treatment instrument for endoscope 10 generally includes, as shown in Figure 1, an insertion section 12 which is inserted into a body, and a hand-side operation section 14 connected to the insertion section 12, and the insertion section 12 is configured with a non-conductive and flexible sheath 16, a conductive wire 18 which is inserted into the sheath 16, and a treatment section 20 mounted to a distal end of the flexible sheath 16. The distal end of the wire 18 is connected to the treatment section 20, and the proximal end of the wire 18 is connected to a connector 22 of the hand-side operation section 14. The connector 22 is electrically connected to a high-frequency current supply apparatus (not shown) for supplying a high frequency current. The hand-side operation section 14 has a grasping portion 24 with an operation button 26, so that a press-down of the operation button 26 causes a high frequency current to be applied to the wire 18. The operation of the treatment instrument for endoscope 10 configured as described above is started by a grasping of the grasping portion 24 of the hand-side operation section 14 and an insertion of the insertion section 12 into a clamp channel (not shown) of the endoscope.

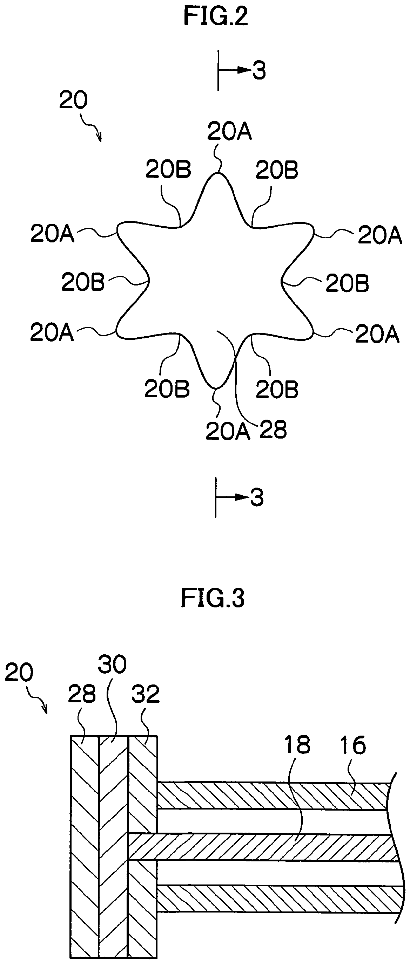

Figure 2 is a front view showing the distal end surface of the treatment section 20 of Figure 1 which is seen in the direction of the arrow A, and Figure 3 is a cross sectional view of the treatment section 20 taken along the 3-3 line of Figure 2.

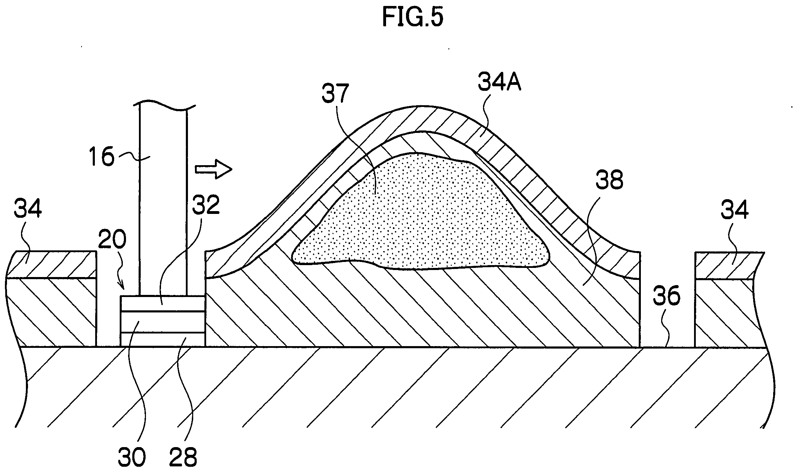

As shown in Figure 2, the treatment section 20 is formed into a gear-like shape. That is, the treatment section 20 has a plurality of alternate peaks 20A, 20A ... and valleys 20B, 20B ... between the peaks in the circumferential direction thereof. Each of the peaks 20A has a round distal end, so that the distal end, even when pressed against a proper muscular layer 36 (see Figure 5), does not cut the proper muscular layer 36.

As shown in Figure 3, the treatment section 20 is configured with a conductor 30 formed of a metal plate and the like, and non-conductors (insulating portions) 28, 32 formed of a non-conductive material such as ceramic and plastic. The conductor 30 and the non-conductors 28, 32 are formed into a common gear shape, so that when superimposed, the side surfaces are coplanar. The configuration makes the side surface of the treatment section 20 have a sandwich structure with the non-conductor 28, 32 sandwiching the strip-shaped conductor 30 therebetween, and the strip-shaped conductor 30 extending around the side surface of the treatment section 20.

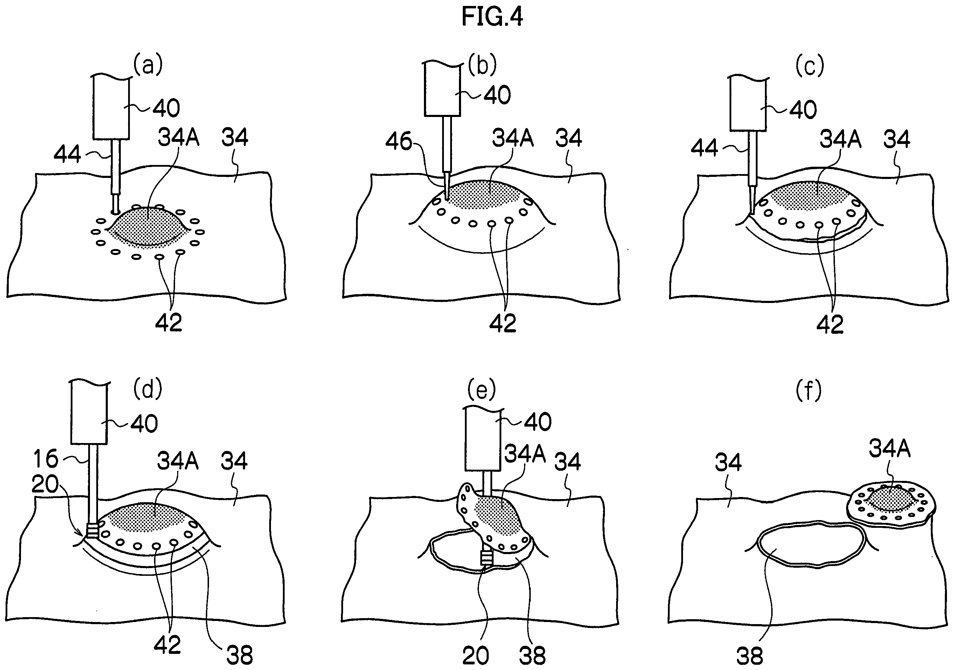

The non-conductor 28 at the distal end side has a thickness which is preferably 0.1 to 2 mm, and more preferably 0.5 to 1 mm, in order to prevent the conductor 30 from contacting the proper muscular layer 36 (see Figure 4) without fail and also cause the conductor 30 to contact a submucosal layer 38 without fail. The non-conductor 32 at the distal end side has a thickness that is preferably 0.1 to 2 mm, and more preferably 0.5 to 1 mm, in order to prevent any thermal burn of the mucous membrane 34. The treatment section 20 preferably has a radial size (the maximum diameter) of 1.5 to 3 mm, and of about 2.5 mm when the endoscope has a clamp channel with an inner diameter of 2.8 mm.

Moreover, the conductor 30 preferably has a thickness of 0.2 to 1 mm, more preferably 0.3 to 0.7 mm, and particularly preferably of 0.5 mm. This is based on the experience result from various thicknesses of the conductor 30 which showed that the conductor 30 of a thickness over the range of 0.2 to 1 mm could not cut tissues, and the conductor 30 of a thickness below 0.3 mm was dull, and the conductor 30 of a thickness above 0.7 mm caused some burn on tissues.

The above conductor 30 is electrically connected to the wire 18. The wire 18 is electrically connected to the connector 22 of Figure 1 as described above, and the connection of the connector 22 to the high-frequency current supply apparatus (not shown) allows a high frequency current to be applied to the conductor 30. The treatment instrument for endoscope 10 of the first embodiment is of a monopolar-type that has only one of the electrodes at the treatment section 20, with the other electrode (neutral electrode) being mounted to a subject.

Next, a method for performing endoscopic submucosal dissection using the above described treatment instrument for endoscope 10 will be explained below based on Figure 4(a) to Figure 4(f). In the following examples, the mucous membrane 34 has a lesion 34A, and the manipulation for removing the lesion 34A without damaging the proper muscular layer 36 will be described.

First, an observation optical system (not shown) having the endoscope insertion section 41 is used to check the lesion 34A. In the checking, it is recommended that dye such as indigo carmine is distributed through an injection port of the endoscope insertion section 41 to stain the lesion 34A.

Then, as shown in Figure 4(a), marks 43, 43 ... are made around the lesion 34A with predetermined spaces therebetween. The method for making the marks 43 is not limited to any particular one, but for example, a high-frequency knife 45 having a distal end of a needle shape may be used. The high-frequency knife 45 is the one made by inserting a thin metal lead into an insulating tube and protruding the distal end of the metal lead from the distal end of the insulating tube by a predetermined length, and so the protruded portion of the metal lead functions as an electrode for incision or cutting of a wall inside of a body when applied with a high frequency current.

Next, as shown in Figure 4(b), an injection needle 47 is inserted into the clamp channel of the endoscope insertion section 41, which is delivered out of the distal end of the clamp channel. Then, the injection needle 47 is used for injection (local injection) of a chemical 37 (see Figure 5) into the submucosal layer 38 of the mucous membrane 34 around the lesion 34A. The chemical is usually physiological salt solution, but may be sodium hyaluronate that has a higher viscosity. Such a local injection over the entire part around the lesion 34A causes the entire lesion 34A to be bulged to have a larger volume.

Then, the injection needle 47 is drawn back out of the clamp channel of the endoscope insertion section 41, and the high-frequency knife 45 is inserted into the clamp channel. As shown in Figure 4(c), the high-frequency knife 45 is used for the incision of the mucous membrane 34 at the outer periphery of the lesion 34A along the marks 43. When the incision is completed, as shown in Figure 4(d), the mucous membrane 34 of the lesion 34A shrinks, which reveals the submucosal layer 38.

Next, the high-frequency knife 45 is drawn back out of the clamp channel of the endoscope insertion section 41, and then the treatment instrument for endoscope 10 of the present embodiment is inserted into the clamp channel, and the treatment section 20 is delivered out. Then, the treatment section 20 is inserted into the submucosal layer 38 through the incised part to press the distal end of the treatment section 20 against the proper muscular layer 36 as shown in Figure 5. In the insertion, the treatment section 20 is able to perpendicularly approach a wall of digestive tract. Therefore, the approach of the treatment section 20 can be achieved without changing the posture of the endoscope insertion section 41.

Next, a high frequency current is applied to the conductor 30, and the treatment section 20 is moved in a lateral direction (to the lesion 34A side). This causes the submucosal layer 38 where the conductor 30 contacts to be cut. In the cutting, because the non-conductor 28 is interposed between the conductor 30 and the proper muscular layer 36, the conductor 30 moves along the position offset from the proper muscular layer 36 by the thickness of the non-conductor 28, which prevents the proper muscular layer 36 from being in contact with and being cut by the conductor 30.

The lateral movement of the treatment section 20 (to the inner side of the incised part) causes the submucosal layer 38 to be cut, and the lesion 34A to be gradually removed from the submucosal layer 38 as shown in Figure 4(e). This results in the cutoff of the lesion 34A as shown in Figure 4(f).

As described above, according to the present embodiment, because the side surface of the treatment section 20 have a sandwich structure in which the non-conductors 28 and 32 sandwich the strip-shaped conductor 30 therebetween, only the submucosal layer 38 is cut without fail with no cutting of the proper muscular layer 36.

In addition, according to the present embodiment, because the treatment section 20 is formed into a gear-like shape having the alternate peaks 20A and valleys 20B in the circumferential direction thereof, in the lateral movement of the treatment section 20, the fibrous submucosal layer 38 is likely to be hooked by the treatment section 20, resulting in an efficient cutting of the submucosal layer 38.

Moreover, according to the present embodiment, because the strip-shaped conductor 30 is formed to extend around the side surface of the treatment section 20, the treatment section 20 is provided with cutting ability at the entire side surface thereof, thereby the treatment section 20 cuts the submucosal layer 38 in any lateral direction the treatment section 20 moves.

In addition, according to the present embodiment, because the treatment section 20 perpendicularly approaches a wall of digestive tract, the approach of the treatment section 20 can be done while a lesion is squarely observed. Furthermore, according to the present embodiment, because a cutting is performed by moving the treatment section 20 in a lateral direction, the cutting can be done while a lesion is squarely observed.

In the above described embodiment, the treatment section 20 has six peaks 20A and six valleys 20B, but the numbers of the peaks 20A and the valleys 20B is not limited to the above example, and may be 3 to 5, or 7 or more. Furthermore, the shape of the treatment section 20 is not limited to the gear-like shape, and the treatment section 20 may be a circular plate, an elliptical plate, a polygonal plate such as a triangular plate, a sphere, or a star shape cut from a sphere.

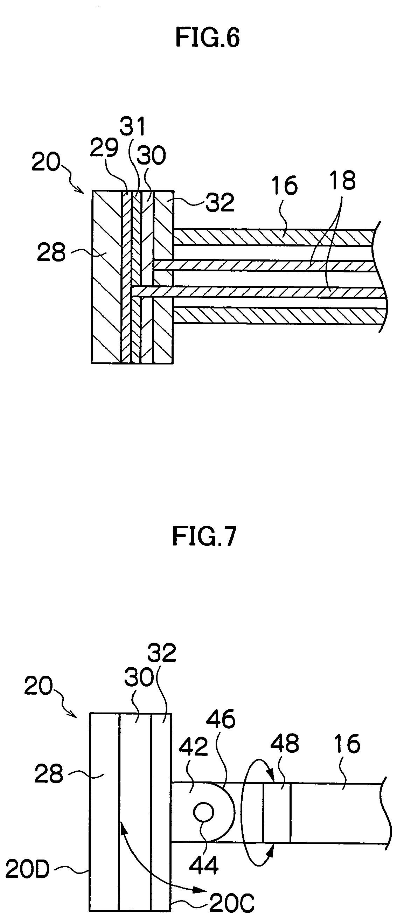

The above described embodiment is illustrated using a monopolar-type that has one of the electrodes at treatment section 20, but the present invention may be applied to a bipolar-type having both of the electrodes at treatment section 20. Figure 6 is a side cross sectional view showing a bipolar type treatment instrument for endoscope. The treatment section 20 shown in Figure 6 is configured by superimposing the non-conductor 28, the conductor 29, the non-conductor 31, the conductor 30, and the non-conductor 32 from the distal end side thereof. Each of the conductors 29 and 30 and each of the non-conductors 28, 31, and 32 are formed into the same planar shape, so that the superimposed conductors and non-conductors have coplanar side surfaces. Therefore, the side surfaces of the treatment section 20 have a multi-layered sandwich structure in which the strip-shaped conductor 29 is sandwiched between the non-conductors 28 and 31, and the strip-shaped conductor 30 is sandwiched between the non-conductors 31 and 32. The conductors 29 and 31 are connected to the wires 18 and 18 respectively, and, via the wires 18, are further connected to the high-frequency current supply apparatus. The wires 18 are individually covered to prevent shorting due to any contact therebetween.

In the case of the bipolar-type treatment instrument for endoscope configured as described above, when the submucosal layer 38 contacts the side surface of the treatment section 20, a high frequency current flows between the conductors 29 and 30 via the submucosal layer 38, which cuts the submucosal layer 38.

The above described first embodiment is an example where the treatment section 20 is fixed at the distal end of the insertion section 12 (sheath 16), but as shown in the following description, the treatment section 20 may be fixed via a swing mechanism so that the postures of the treatment section 20 can be changed.

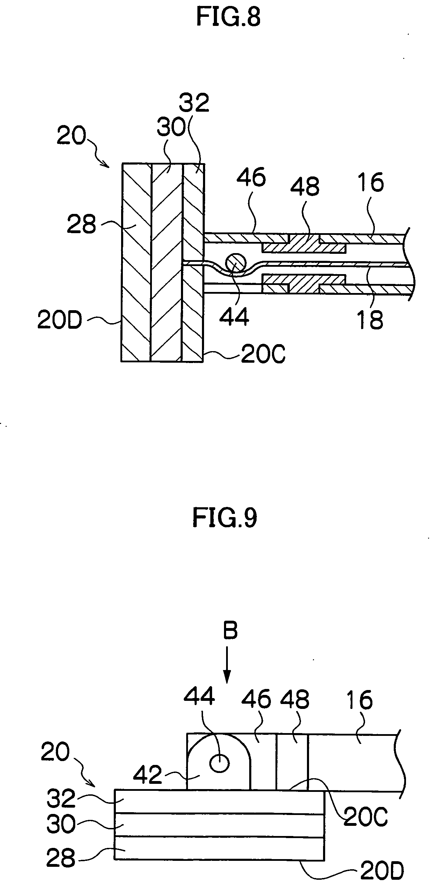

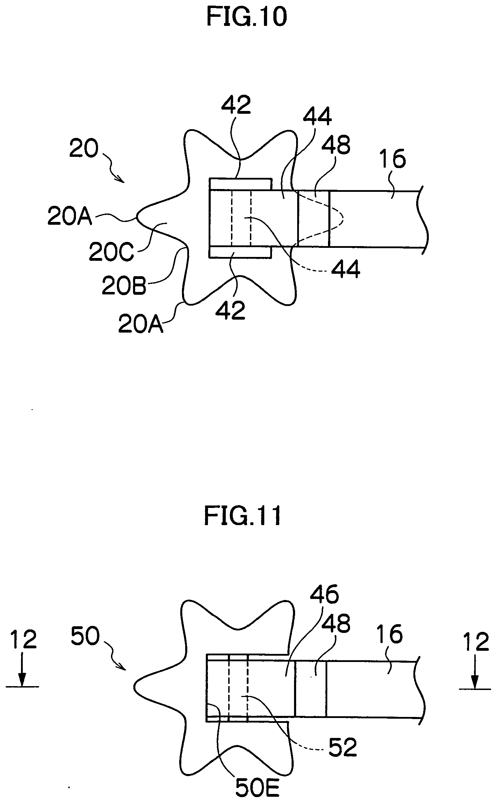

Figure 7 is a side view showing the distal end portion of a treatment instrument for endoscope of a second embodiment, Figure 8 is a side cross sectional view showing the cross section of Figure 7, Figure 9 is a side view showing the treatment section of Figure 7 in another posture, and Figure 10 is a view of the treatment section of Figure 9 seen in the direction B.

As shown in these figures, in the second embodiment, the treatment section 20 has a proximal end surface 20C from which a pair of bearings 42, 42 are protruded, and the bearings 42, 42 support a shaft 44. To the shaft 44, a first sleeve 46 is rotatably coupled at the distal end portion thereof, and to the proximal end portion of the first sleeve 46, a second sleeve 48 is engaged at the distal end portion thereof. The first sleeve 46 and the second sleeve 48 are coaxially arranged and coupled with each other, and also are coupled to relatively rotate about the central axes of the sleeves. To the proximal end of the second sleeve 48, the sheath 16 is coupled.

In the treatment instrument for endoscope configured as described above, the treatment section 20 is rotatably supported via different two rotation shafts, and the postures of the treatment section 20 can be changed as needed. That is, a rotation of the treatment section 20 relative to the first sleeve 46 causes the posture of the treatment section 20 to be inclined with respect to the insertion direction, and a rotation of the first sleeve 46 relative to the second sleeve 48 causes the treatment section 20 to be rotated around the insertion direction. Therefore, according to the second embodiment, the postures of the treatment section 20 can be changed as needed. The configuration allows the distal end surface 20D of the treatment section 20 to follow the shape of the proper muscular layer 36, and the distal end surface 20D of the treatment section 20 to be constantly in contact with the proper muscular layer 36. As a result, the prevention of any contact of the conductor 30 of the treatment section 20 with the proper muscular layer 36 is further ensured.

The above described second embodiment is an example where the postures of the treatment section 20 are passively changed, but the present invention is not limited to the example, and the postures of the treatment section 20 may be actively changed. For example, a wire (not shown) may be coupled at the distal end thereof to the non-conductor 32 of the treatment section 20, and the wire may be extended to the hand-side operation section 14, so that the treatment section 20 can be rotated in accordance with an advancing/retracting operation of the wire.

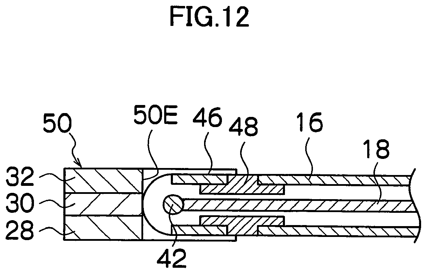

Figures 11 and 12 show a treatment section for endoscope having a configuration which is different from the above described second embodiment. As shown in these figures, a treatment section 50 has a side surface which is notched from one side to form a groove 50E. The groove 50E is provided with a shaft 52 by which the first sleeve 46 is rotatably supported. The groove 50E has a width which is slightly larger than the outer diameter of the first sleeve 46, so that when the treatment section 50 is rotated relative to the first sleeve 46, the first sleeve 46 is inserted into the groove 50E. The shaft 52 is formed of a conductive material such as metal. The shaft 52 is connected to the conductor 30, and to the shaft 52, the wire 18 is connected.

According to the treatment instrument for endoscope configured as described above, a rotation of the treatment section 50 relative to the sleeve 46 causes the first sleeve 46 to be disposed in the groove 50E of the treatment section 50. As a result, the treatment section 50 has a reduced thickness, which enhances the insertability of the endoscope into the clamp channel.

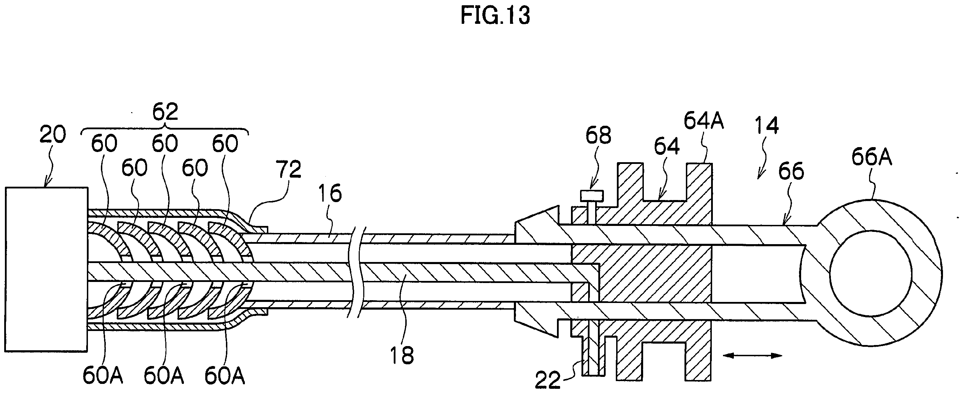

The configuration of the swing mechanism of the treatment sections 20 and 50 is not limited to the above described embodiment, and there are various embodiments for the configuration. For example, as shown in Figure 13, the treatment section 20 may be supported via a bending portion 62 that has a plurality of cup members 60, 60 ... The cup members 60 of Figure 13 have a hole 60A formed herein, into which the wire 18 are inserted. The wire 18 is coupled at the distal end thereof to the treatment section 20, and is also coupled at the proximal end to a slider 64 of the hand-side operation section 14. The slider 64 is slidably supported by the body 66 of the hand-side operation section 14, and so an operation of a locking screw 68 provided at the slider 64 causes a lock/unlock between the slider 64 and the body. The slider 64 has a flange 64A on which a surgeon's forefinger and middle finger are placed, and the body 66 has a ring portion 66A formed at the proximal end thereof in which the surgeon's thumb is placed.

The sheath 16 has a proximal end that is fixedly attached to the body 66 of the hand-side operation section 14, and a distal end which is fixedly attached to the cup member 60 on the most proximal end side. The sheath 16 has a proper rigidity, so that the sheath 16 is not broken or crashed even when the slider 64 slides to the proximal end side to increase the tension of the wire 18.

The bending portion 62 is covered with a covering tube 70 that is formed of a soft material such as rubber. The covering tube 70 has a distal end that is fixedly attached to the treatment section 20, and a proximal end that is attached to the proximal end of the sheath 16.

In the treatment instrument for endoscope configured as described above, a sliding of the slider 64 of the hand-side operation section 14 to the distal end side relative to the body 66 reduces the tension of the wire 18, and decreases the friction between the cup members 60. Thus, the bending of the bending portion 62 is under control.

On the contrary, a sliding of the slider 64 to the proximal end side relative to the body 66 increases the tension of the wire 18, and increases the friction between the cup members 60. Thus, the shape of the bending portion 62 is fixed then.

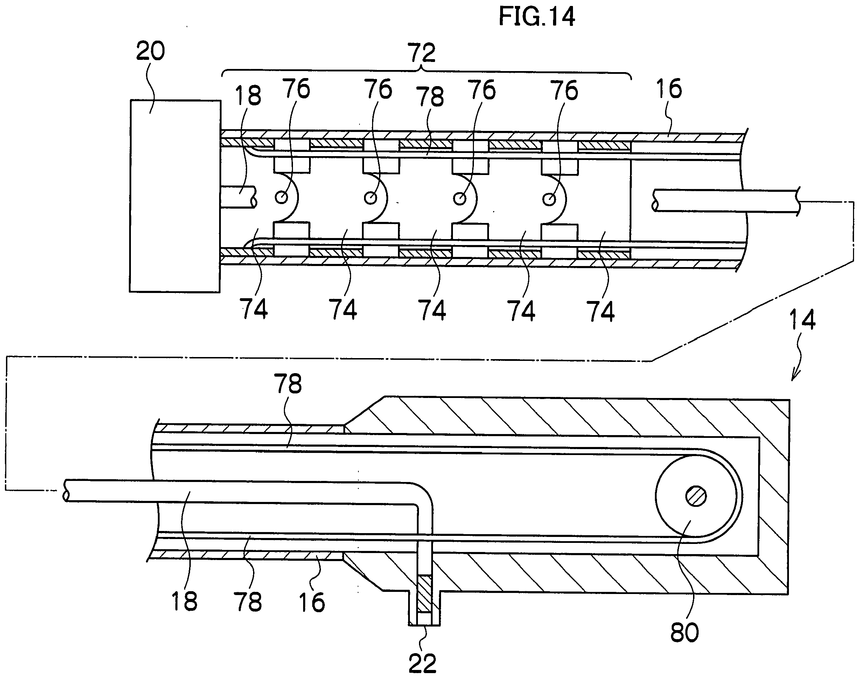

Figure 14 is a schematic view showing a treatment instrument for endoscope that has a swing mechanism of another configuration. The treatment instrument for endoscope of Figure 14 includes the treatment section 20 that is supported by a bending portion 72 having a plurality of cylindrical nodal rings 74, 74 ..., and the nodal rings 74 are rotatably coupled to each other by pin 76. Among the plurality of nodal rings 74, the nodal ring 74 at the distal end is fixedly attached to the treatment section 20, and to the nodal ring 74, operation wires 78, 78 are fixed at the distal ends thereof. The operation wires 78 are inserted into the sheath 16 to be wound around a pulley 80 at the hand-side operation section 14. Thus, a rotation of the pulley 80 using a knob (not shown) or the like causes the operation wires 78 to be advanced or retracted, resulting in the rotation of the nodal rings 74 for a bending operation of the bending portion 72. According to the treatment instrument for endoscope configured as described above, the bending portion 72 is bendable and so the posture of the treatment section 20 is under control. Thus, the approach of the treatment section 20 to the submucosal layer 38 is facilitated, and also the cutting of the submucosal layer 38 is facilitated.

In Figure 14, the bending structure for bending in two directions (upward and downward) is shown, but the bending directions are not limited to those, and the structure may be bendable in four upward, downward, leftward, and rightward directions. In addition to the above described embodiment, the postures of the treatment section 20 may be changed by using a rack and pinion, or by supporting the treatment section 20 by a linear member formed of a shape-memory alloy and Joule-heating and deforming the linear member by heat, for example.

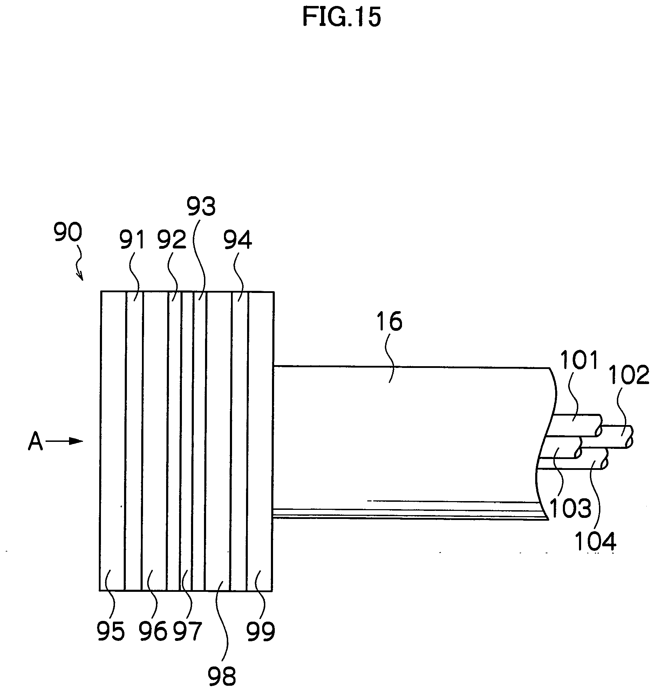

Next, a treatment instrument for endoscope of a third embodiment will be explained below. Figure 15 is a side view showing the characteristic parts of a treatment instrument for endoscope of the third embodiment. As shown in Figure 15, a treatment section 90 of the third embodiment is configured by superimposing four conductors 91, 92, 93 and 94, and five non-conductors 95, 96, 97, 98 and 99. That is, the treatment section 90 is configured by superimposing the non-conductor 95, the conductor 91, the non-conductor 96, the conductor 92, non-conductor 97, the conductor 93, the non-conductor 98, the conductor 94, and the non-conductor 99 from the distal end side thereof. Each of the conductors 91 to 94 and each of the non-conductors 95 to 99 are formed into the same planar shape (for example, a gear-like shape) respectively when seen in the direction A, so that the superimposed conductors and non-conductors are completely aligned with each other. Therefore, the side surfaces of the treatment section 90 have a multi-layered sandwich structure with the conductors 91 to 94 being sandwiched between the non-conductors 95 to 99.

The non-conductor 97 disposed between the central conductors 92 and 93 has a thickness smaller than those of the other non-conductors 96 and 98. Therefore, the distance between the central conductors 92 and 93 is smaller than that between the conductors 91 and 92 or that between the conductors 93 and 94, on both sides thereof.

Each of the conductors 91 to 94 is connected to metal leads 101 to 104 respectively, and the metal leads 101 to 104 are connected to an external high-frequency current supply apparatus (not shown) via the connector 22 of the hand-side operation section 14. The high-frequency current supply apparatus selects two metal leads 101 to 104 to which a current is applied,out of the four metal leads 101 to 104, and applies a different high frequency current in response to the selected metal leads 101 to 104. For example, the high-frequency current supply apparatus selects the conductors 91 and 92, and also applies a high frequency current for coagulation using the conductors 91 and 92 as one pair of electrodes. Similarly, the high-frequency current supply apparatus selects the conductors 93 and 94, and also applies a high frequency current for coagulation using the conductors 93 and 94 as one pair of electrodes. Furthermore, the high-frequency current supply apparatus selects the conductors 92 and 93, and also applies a high frequency current for coagulation using the conductors 92 and 93 as one pair of electrodes. This allows the submucosal layer 38 that contacts the part between the conductors 91 and 92 and the submucosal layer 38 that contacts the part between the conductors 93 and 94 to be coagulated, while the submucosal layer 38 that contacts the part between the central conductors 92 and 93 is allowed to be cut. The high-frequency current supply apparatus repeats the coagulation process with the selection of the conductors 91, 92 and the conductors 93, 94, and the cutting process with the selection of the conductors 92 and 93 in very short cycles. This makes the coagulation process and the cutting process apparently done at the same time.

When the treatment instrument for endoscope configured as described above moves the treatment section 90 in a lateral direction and the submucosal layer 38 is pressed against the side surface of the treatment section 90, the submucosal layer 38 that contacts the part between the conductors 91 and 92 on the distal end side and the submucosal layer 38 that contacts the part between the conductors 93 and 94 on the proximal end side are coagulated, while the submucosal layer 38 that contacts the part between the central conductors 92 and 93 is cut. Therefore, the part between the coagulated parts is cut, which considerably reduces a resulting amount of bleeding. Especially in the present embodiment, coagulated tissues are left on the proper muscular layer 36 side as well as on the mucous membrane 34 side, thereby a resulting amount of bleeding can be significantly reduced.

The third embodiment is a bipolar-type treatment instrument performing a coagulation process only on the tissues between the electrodes (between the conductors), which enables a control to narrow the tissue area for coagulation. Therefore, the tissue area which may be damaged is narrowed, and the tissue damage caused by coagulation can be minimized.

In the above described third embodiment, both of the distal end side (the proper muscular layer 36 side) and proximal end side (the mucous membrane 34 side) of a cutting is processed for coagulation, but the positions for coagulation are not limited to those, and for example, a high frequency current for coagulation may be applied to the part between the conductors 91 and 94 so as to coagulate the entire area. Alternatively, a high frequency current for coagulation may be applied only to the conductors 91 and 92, so as to coagulate only the distal end side of the incision position.

Also, in the above described third embodiment, the four conductors 91 to 94 are provided, but the number of the conductor is not limited to four, and any number equal to three or more of conductors is needed for a bipolar type. In the case, two conductors including the conductor on the most distal end side may be selected for applying a high frequency current for coagulation, and two conductors except the conductor on the most distal end side may be selected for applying a high frequency current for cutting. For example, in a case with three conductors, it is recommended that the conductor on the most distal end side and the conductor on the most proximal end side are selected for applying a high frequency current for coagulation, and the conductor on the most proximal end side and the central conductor are selected for applying a high frequency current for cutting.

To the contrary, for a monopolar type, any number equal to two or more of conductors is needed. In the case, at least one of the conductors including the conductor on the most distal end side may be selected for applying a high frequency current for coagulation, and one of the conductors except the conductor on the most distal end side may be selected for applying a high frequency current for cutting. For example, in a case with three conductors, the conductors on the distal end side and on the proximal end side may be selected for applying a high frequency current for coagulation, and the central conductor may be selected for applying a high frequency current for cutting. All of the three conductors may be selected, or only the conductor on the distal end side may be selected for a high frequency current for coagulation.

In addition, in a case of a monopolar type with two conductors, both of the conductors (or only the conductor on the distal end side) may be selected for applying a high frequency current for coagulation, and the conductor on the proximal end side may be selected for applying a high frequency current for cutting.

In the above described first to the third embodiments, the conductors and the non-conductors that have the same planar shape are superimposed to form a treatment section, but the configuration of the treatment section is not limited to this, and any sandwich structure can be used as long as a strip-shaped conductor is formed at a side surface of the treatment section and the conductor is sandwiched between non-conductors. Therefore, the conductor may be formed into a tubular shape (donut shape) and configured to be exposed at a side surface.

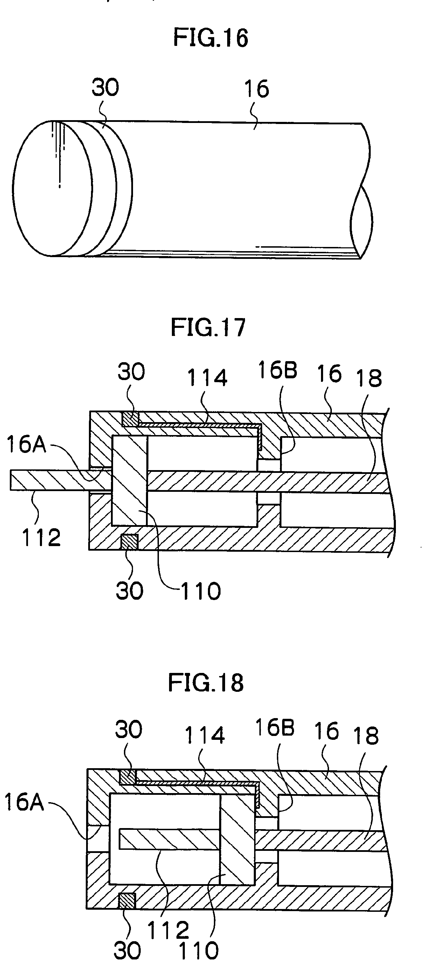

In addition, as shown in Figure 16, the conductor 30 may be provided on the outer peripheral surface of the sheath 16 that is a non-conductor. In the case, the conductor 30 may be disposed at a position separated from the distal end of the sheath 16 by a predetermined distance, and also may be formed to extend around the circumference of the sheath 16 in a strip shape. The configuration enhances the insertability of the endoscope into the clamp channel.

Figures 17 and 18 are cross sectional views showing a treatment instrument for endoscope which has a conductor 30 on the outer peripheral surface of the sheath 16 similar to Figure 16, and is provided with a function of a high-frequency knife. As shown in these figures, the wire 18 is inserted into the inside of the sheath 16, and a metallic disc 110 is attached to the distal end of the wire 18, and furthermore, a needle knife 112 is attached to the disc 110. To the proximal end of the wire 18, a slider 64 of the hand-side operation section 14 (see Figure 13) is attached, so that an advancing/retracting operation of the wire 18 can be manipulated.

The distal end surface of the sheath 16 has a hole 16A formed therein, and an advancing operation of the wire 18 to the distal end side causes a needle knife 112 to be protruded out of the hole 16A as shown in Figure 17. In the protrusion, the disc 110 contacts the distal end surface of the sheath 16, which limits the protrusion amount of the needle knife 112. Also, a retracting operation of the wire 18 to the proximal end side causes a needle knife 112 to be drawn back through the hole 16A into the sheath 16 as shown in Figure 18.

The sheath 16 is also provided with a projection 16B at the inner peripheral surface thereof, so that when the needle knife 112 is completely received in the sheath 16, the disc 110 contacts the projection 16B. The projection 16B has a metal lead 114 attached thereto that is connected to the conductor 30 provided at the outer peripheral surface of the sheath 16, and a contact of the disc 110 with the projection 16B causes the conductor 30 to be electrically connected to the wire 18.

According to the treatment instrument for endoscope configured as described above, the needle knife 112 is protruded by an advancing operation of the wire 18 so as to be used as a high-frequency knife, which allows the marking process and the cutting process of the mucous membrane 34 to be performed by the needle knife 112. Also, a retracting operation of the wire 18 causes a high frequency current to be applied to the conductor 30 provided at the outer peripheral surface of the sheath 16, which can be used as the means for cutting the submucosal layer 38.

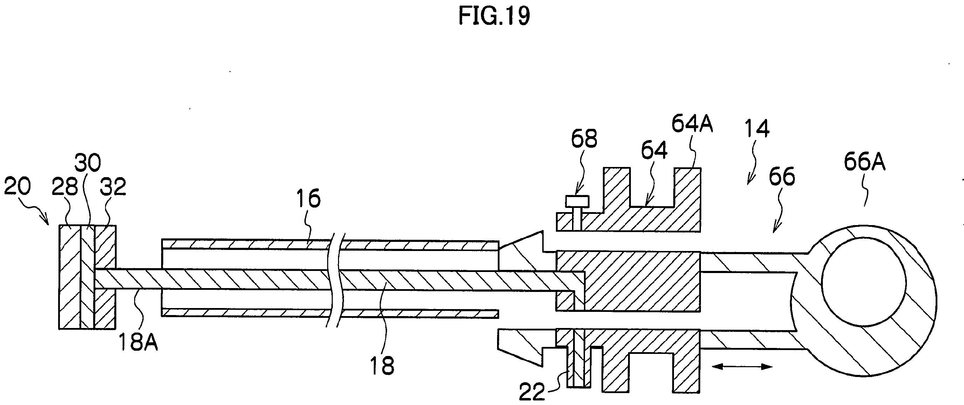

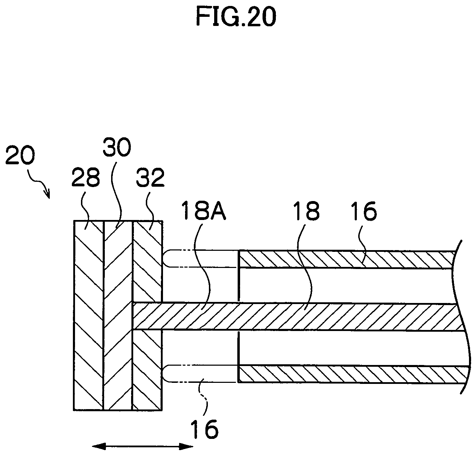

Next, a treatment instrument for endoscope of a fourth embodiment will be explained below. Figure 19 is a cross sectional view showing the treatment section 20 of a treatment instrument for endoscope of the fourth embodiment, and Figure 20 is a cross sectional view showing the treatment section of Figure 19.

The treatment section 20 of the fourth embodiment shown in these figures is formed into a gear-like shape, and also is configured to have a sandwich structure with the non-conductor 28, 32 sandwiching the conductor 30 therebetween, similar to the treatment section 20 of the first embodiment shown in Figures 1 to 3.

The conductor 30 is connected to the wire 18 for the connection to a high-frequency source (not shown), and the wire 18 is configured to be movable forward and backward relative to the sheath 16. That is, the wire 18 is coupled at the proximal end thereof to the slider 64 of the hand-side operation section 14, and the slider 64 is slidably supported by the body 66. Meanwhile, the insulative sheath 16 is coupled to the body 66 of the hand-side operation section 14 at the proximal end thereof, so that a sliding of the slider 64 relative to the body 66 causes the wire 18 (i.e., the treatment section 20) to be operated forward and backward relative to the sheath 16. Therefore, a backward sliding of the slider 64 relative to the body 66 causes the treatment section 20 to be retracted relative to the sheath 16, and on the contrary, a forward sliding of the slider 64 relative to the body 66 causes the treatment section 20 to be advanced relative to the sheath 16.

When the treatment section 20 is advanced, as shown in Figures 19 and 20 by the solid lines, the distal end portion 18A of the wire 18 is exposed. Therefore, in the exposed state, an application of a high frequency current to the wire 18 enables a cutting with the distal end portion 18A of the wire 18. In the application, the high frequency current is also applied to the conductor 30, thereby a cutting can be also performed with the conductor 30 exposed at the side surface of the treatment section 20.

When the treatment section 20 is retracted, a shown in Figure 20 by the two-dot chain line, the distal end of the sheath 16 contacts the proximal end surface of the treatment section 20, and the wire 18 is received in the sheath 16 to be in a non-exposed state. Therefore, when a high frequency current to the wire 18 is applied in the state, the cutting is performed only with the conductor 30 at the side surface of the treatment section 20.

The hand-side operation section 14 shown in Figure 19 has the same configuration as that of the hand-side operation section 14 shown in Figure 13, which will not be explained below in detail. The configuration for the advancement/retraction of the treatment section 20 relative to the sheath 16 is not limited those above described embodiments, and can be changed in various manners.

The treatment instrument for endoscope configured as described above performs a cutting using the distal end portion 18A of the wire 18 by advancing the treatment section 20 relative to the sheath 16, which allows the treatment instrument for endoscope to be used for incision of the mucous membrane 34. That is, in the incision procedure of the mucous membrane 34 shown in Figure 4(c), instead of the high-frequency knife 45, the treatment instrument for endoscope of the fourth embodiment may be used for incision of the mucous membrane 34.

In the incision of the mucous membrane 34, first, a window is formed in the mucous membrane 34 by a precut or the like, and the treatment section 20 is inserted into the window from the distal end surface thereof. Then, with the distal end portion 18A of the wire 18 being exposed, a high frequency current is applied to the wire 18 while the treatment section 20 is laterally moved. The movement causes the distal end portion 18A of the wire 18 to contact the mucous membrane 34, thereby the mucous membrane 34 is incised. In the incision, the treatment section 20 is only laterally moved without changing its posture to cut the mucous membrane 34, which facilitates the cutting of the mucous membrane 34. In addition, the high frequency current is also applied to the conductor 30 that is exposed at the side surface of the treatment section 20, thereby the cutting of the submucosal layer 38 can be performed at the same time as the cutting of the mucous membrane 34. Furthermore, because the non-conductor 28 is provided at the distal end side of the treatment section 20, the cutting of the mucous membrane 34 and the submucosal layer 38 can be safely performed without damaging the proper muscular layer 36.

After the incision of the mucous membrane 34, the treatment section 20 is retracted relative to the sheath 16, and the wire 18 is received in the sheath 16. Then, when the wire 18 is in a non-exposed state, a high frequency current is applied to the conductor 30 while the treatment section 20 is laterally moved for the removal of the submucosal layer 38. This allows the present embodiment to perform the incision of the mucous membrane 34 and the removal of the submucosal layer 38 without replacing the treatment instrument for endoscope, as the result of that the operating efficiency can be considerably enhanced and the burden on patient can be significantly reduced.

In the above described fourth embodiment, the exposed state and the non-exposed state of the distal end portion 18A of the wire 18 is selectable, but as will be described later, the exposed state and the non-exposed state of the bar-shaped electrode connected to the distal end of the wire 18 may be set to be selectable. Alternatively, another embodiment may be used in which the distal end portion 18A of the wire 18 and the bar-shaped electrode are constantly exposed.

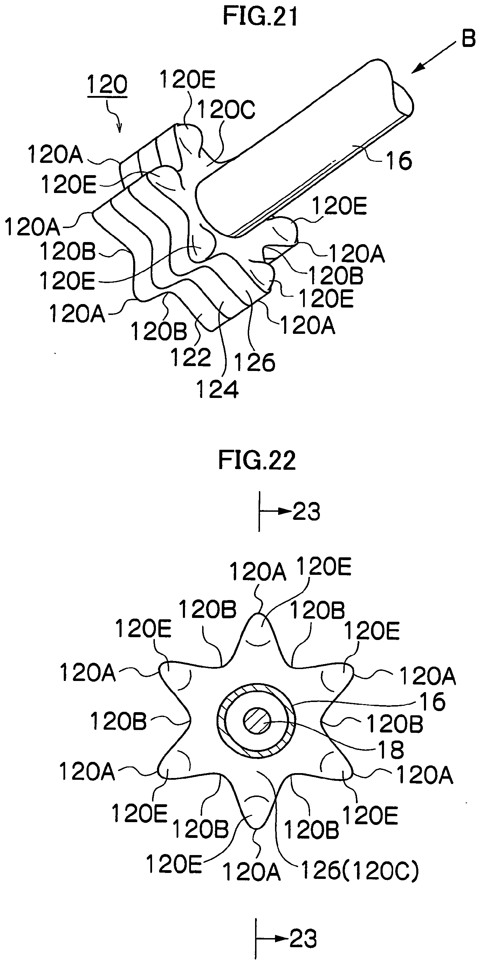

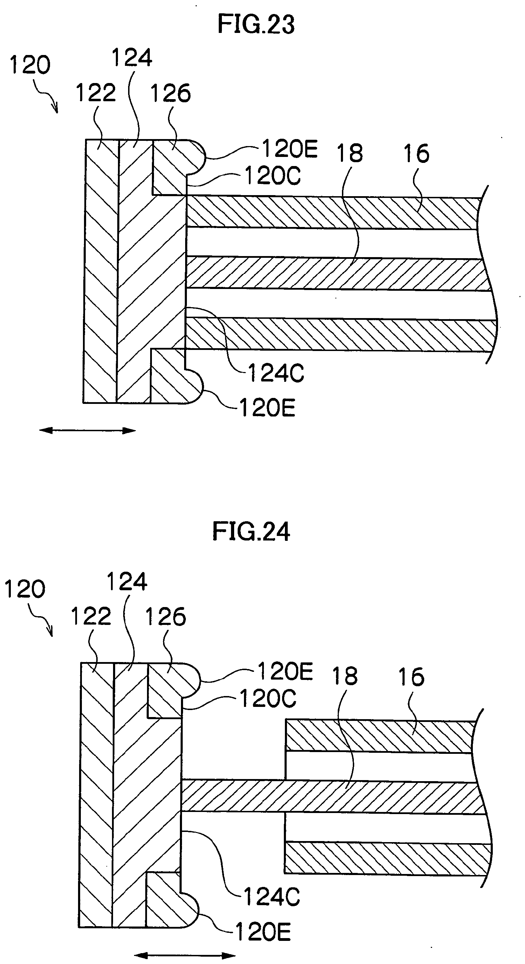

Next, a treatment instrument for endoscope of a fifth embodiment will be explained below. Figure 21 is a perspective view showing a treatment section 120 of a treatment instrument for endoscope of the fifth embodiment, Figure 22 is a plan view of the treatment section 120 seen in the direction of the arrow B, Figure 23 is a cross sectional view taken along the 23-23 line of Figure 22, and Figure 24 is a cross sectional view showing a situation where the treatment section 120 of Figure 23 is advanced relative to a sheath 16.

The treatment section 120 shown in these figures is formed into a gear-like shape, and has alternate peaks 120A, 120A ..., and valleys 120B, 120B ...between the peaks in the circumferential direction thereof, similar to the treatment section 20 of the first embodiment shown in Figures 1 to 3. Also, the treatment section 120 is configured to have a sandwich structure in which non-conductors 122, 126 sandwich a conductor 124 therebetween, and the strip-shaped conductor 124 is formed to be extended along the side surface of the treatment section 120.

Each of the peaks 120A has a proximal end surface 120C (i.e., the non-conductor 126) that has a projection 120E formed thereon. Each projection 120E is disposed on the outer most peripheral side and is formed to protrude as a generally semi-spherical shape, so that the submucosal layer 38 can be hooked by the projection 120E.

Also, the treatment section 120 is provided to be movable forward and backward relative to the sheath 16, similar to the fourth embodiment, so as to be retracted relative to the sheath 16 as shown in Figure 23, and be advanced retracted relative to the sheath 16 as shown in Figure 24.

As shown in Figure 24, the conductor 124 is exposed at the side surface of the treatment section 120 in a strip shape, and is also exposed on the proximal end surface 120C side of the treatment section 120, which forms a proximal end side exposed surface 124C. The proximal end side exposed surface 124C is disposed on the central side of the treatment section 120, and is exposed when the treatment section 120 is advanced relative to the sheath 16 as shown in Figure 24, and is received in the sheath 16 to be in a non-exposed state when the treatment section 120 is retracted relative to the sheath 16 as shown in Figure 23.

According to the fifth embodiment configured as described above, because the projection 120E is formed on the proximal end surface 120C of the treatment section 120 is formed, a movement of the treatment section 120 to the proximal end side causes the fibrous submucosal layer 38 to be hooked by the projection 120E. The hooked submucosal layer 38 is collected to the central side of the treatment section 120, which is brought into contact with the proximal end side exposed surface 124C or the distal end of the wire 18 portion 18A to be cut. That is, a "draw-cut" can be performed in which while the treatment section 120 is moved to the proximal end side, the submucosal layer 38 is cut. As the result, the removal efficiency of submucosal layer 38 can be enhanced, and the burden on patient can be reduced due to the time saving in the removal operation.

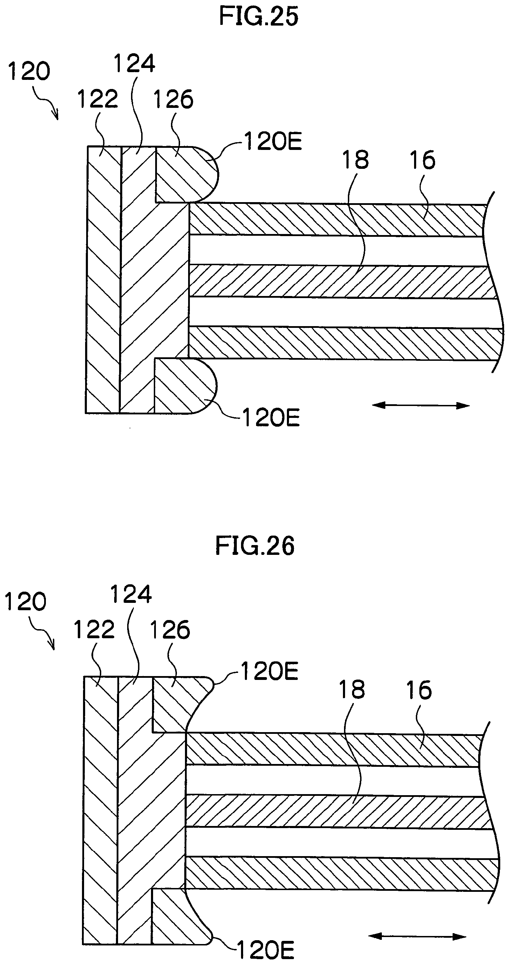

In the above described fifth embodiment, the projections 120E are formed only on the outer peripheral side of the treatment section 120, but the projections 120E may be formed to protrude from the entire part outside of the sheath 16, as shown in Figure 25. The shape of the projections 120E is not limited to the semi-spherical one, and the submucosal layer 38 may have any shape that is able to hook the submucosal layer 38, and for example, the treatment section 120 may be protruded in a generally cylindrical shape or a generally conical shape. Alternatively, as shown in Figure 26, the projections 120E may be formed to have a shape in which an amount of protrusion is the maximum at the outward position thereof, and gradually decreases toward the inward position thereof. Furthermore, the projections 120E may be formed by forming a groove or a recess in the proximal end surface 120C of the treatment section 120.

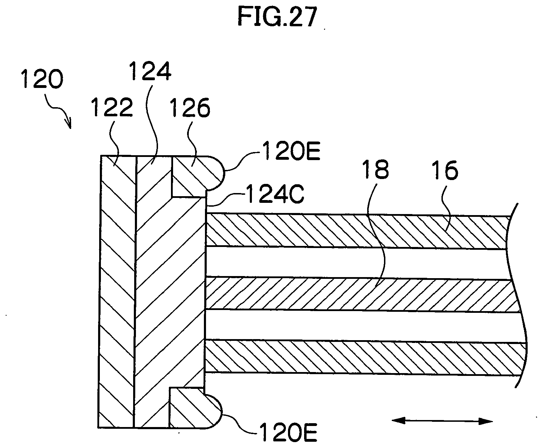

In the above described fifth embodiment, the proximal end side exposed surface 124 of the conductor 124 is exposed when the treatment section 120 is advanced relative to the sheath 16, but the proximal end side exposed surface 124 may be constantly exposed. For example, the treatment section 120 shown in Figure 27 has a conductor 124 formed to have a larger size than that of the sheath 16, so that the proximal end side exposed surface of the conductor 124 is constantly exposed at the outside of the sheath 16. Thus, a cutting of the submucosal layer 38 can be performed at any time without an advancing/retracting operation of the treatment section 120. In the case of the treatment section 120 of Figure 27, the embodiment in which the treatment section 120 is fixed relative to the sheath 16 may be possible.

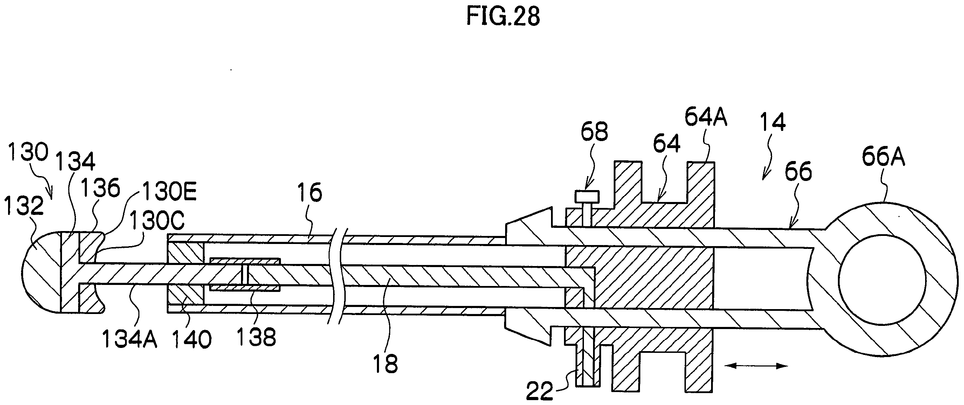

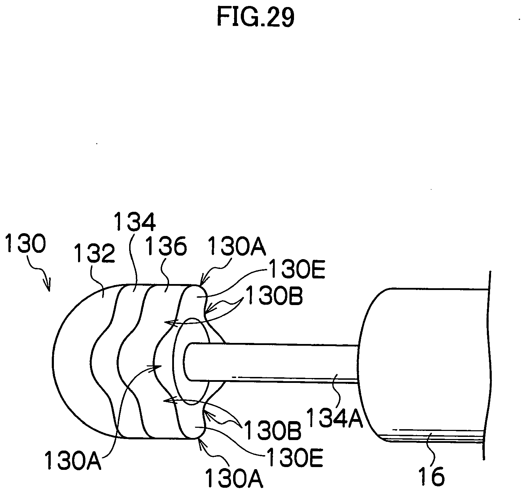

Next, a treatment instrument for endoscope of a sixth embodiment will be explained below. Figure 28 is a cross sectional view showing a treatment instrument for endoscope of the sixth embodiment, and Figure 29 is a perspective view showing the treatment section 130 thereof.

The treatment section 130 of the sixth embodiment shown in these figures is formed into a gear like shape as seen from the front side (the left side of Figure 28), and has alternate peaks 130A, 130A ..., and valleys 130B, 130B. Also, the treatment section 130 is configured to have a sandwich structure with non-conductors 132, 136 sandwiching a conductor 134 therebetween.

The non-conductor 132 is formed on the distal end side into a round and generally semi-spherical shape (specifically, a gear-like shape cut from a semi sphere). Thus, when the treatment section 130 is laterally moved with the non-conductor 132 being pressed against the proper muscular layer 36, the friction between the non-conductor 132 and the proper muscular layer 36 is reduced, and the smooth movement of the treatment section 130 can be achieved.

The non-conductor 136 on the proximal end side has projections 130E formed on a proximal end surface 130C thereof, similar to the treatment section 120 of the fifth embodiment shown in Figures 21 to 24. The projections 130E are formed to have the peaks 130A that protrudes most at the outer most peripheral side, so that the submucosal layer 38 can be hooked by the projections 130E.

The conductor 134 is exposed at the side surface of the treatment section 130 in a strip-shape, and also protrudes in a bar form from the proximal end surface 130C of the treatment section 130 so as to form a bar-shaped electrode 134A. The bar-shaped electrode 134A has a distal end that is fitted in a connection pipe 138 and is fixed there by welding or brazing. In to the opposite side of the connection pipe 138, the distal end of the wire 18 is fitted, and is fixed there by welding or brazing. The configuration allows the bar-shaped electrode 134A to be connected to the distal end of the wire 18.

The wire 18 is coupled at the proximal end thereof to the slider 64 of the hand-side operation section 14, and the slider 64 is slidably supported by the body 66 of the hand-side operation section 14. To the body 66, the sheath 16 is connected at the proximal end thereof, and a sliding of the slider 64 relative to the body 66 cause the wire (i.e., the treatment section 130) to be operated forward and backward relative to the sheath 16. That is, a backward sliding of the slider 64 relative to the body 66 causes the treatment section 130 to be retracted relative to the sheath 16, and on the contrary, a forward sliding of the slider 64 relative to the body 66 causes the treatment section 130 to be advanced relative to the sheath 16 to the distal end side.

When the treatment section 130 is advanced, the bar-shaped electrode 134A is exposed, and in the exposed state, an application of a high frequency current to the bar-shaped electrode 134A enables a cutting with the bar-shaped electrode 134A. Therefore, the incision of the mucous membrane 34 can be performed using the treatment instrument for endoscope of the sixth embodiment.

When the treatment section 130 is retracted, the distal end of the sheath 16 contacts the proximal end surface of the treatment section 130, and bar-shaped electrode 134A is received in the sheath 16 to be in a non-exposed state. Therefore, the cutting of the conductor 134 and the removal operation of the submucosal layer 38 are performed only with the conductor 134 exposed at the side surface of the treatment section 130.

The distal end portion of the sheath 16 has a collar member 140 formed on the inner side thereof. The collar member 140 is formed of a thermal insulating material such as ceramic, and the collar member 140 prohibits the heat of the bar-shaped electrode 134A from transmitting to the sheath 16. Also, the collar member 140 is formed into a tubular shape having an inner diameter that is slightly larger than the outer diameter of the bar-shaped electrode 134A, which ensures the linear advancement of the treatment section 20 as a result of the guidance of bar-shaped electrodes 134A by the collar member 140. In addition, the collar member 140 also functions to limit the projection (advancement) amount of the treatment section 130, and when the treatment section 130 is advanced relative to the sheath 16, the connection pipe 138 contacts the collar member 140, which limits the advancement.

In the sixth embodiment, the treatment section 130 has the largest outer diameter which is generally equal to the outer diameter of the sheath 16. This allows the treatment instrument for endoscope to be smoothly inserted into the clamp channel of the endoscope insertion section 41 (see Figure 4) without being trapped by the treatment section 130. Alternatively, the embodiment in which the treatment section 130 has a largest outer diameter less than the outer diameter of the sheath 16 is also possible.

According to the sixth embodiment configured as described above, because the treatment section 130 is provided to be movable forward and backward relative to the sheath 16, the bar-shaped electrode 134A can be exposed for the incision of the mucous membrane 34. According to the sixth embodiment, because the treatment section 130 has the projection 130E formed on the proximal end surface 130C (i.e., the non-conductor 136), it is possible to hook and collect the submucosal layer 38 with the projection 130E to the central side for cutting with the bar-shaped electrode 134A, which improves the removal efficiency of the submucosal layer 38.

The embodiments aspects and examples which do not fall within the scope of the claims are provided for illustrative purpose only and do not form part of the present invention. The invention is defined in the claims as follows:

## Claims

1. A treatment instrument for endoscope (10) comprising an insertion section (12) adapted to be inserted into a body, the insertion section comprises a treatment section (20, 120, 130) provided at a distal end of the insertion section (12),
the treatment section (20, 120, 130) comprises a conductor (30,124, 134) configured to be connected to a hight frequency source wherein the treatment section (20, 120, 130) is is configured in a sandwich structure comprising said conductor (30; 124; 134), a distal end side non-conductor (28; 122; 132) provided on the distal end of the treatment section, and a proximal end side non-conductor (32; 126; 136) provided on the other side of the conductor opposite to the distal end side non-conductor; said conductor is provided between the distal end side non-conductor and the proximal end side non-conductor so as to be exposed in a strip-shape at a side surface of the treatment section, and the side surfaces of the conductor and non-conductors are coplanar and
the proximal end side non-conductor is exposed in a circumferentially-extended strip-shape at the side surface of the treatment section, wherein the treatment section (20, 120, 130) is formed to have a gear-like shape in which a plurality of peaks (20A) and valleys (20B) are alternately arranged in a circumferential direction of the treatment section (20, 120, 130) and upon application of a current to the conductor (30, 124, 134) by the high frequency source, the conductor (30, 124, 134) which is exposed in a stripe shape at the side surface of the treatment section is brought into contact with a side of a part of the body to be cut.

2. The treatment instrument for endoscope according to claim 1, wherein
the strip-shaped conductor is formed to extend around the side surface of the treatment section.

3. The treatment instrument for endoscope according to any one of claims 1 to 2, wherein the treatment section is supported via a swing mechanism for controlling a posture of the treatment section.

4. The treatment instrument for endoscope according to any one of claims 1 to 3, wherein the strip-shaped conductor includes at least three conductors that are disposed in parallel to each other and sandwich non-conductors therebetween, and
the treatment instrument for endoscope comprises
a high-frequency current supply apparatus which selects two conductors including one at the most distal end side, out of the at least three conductors to apply a high frequency current for coagulation with using the selected two conductors as a pair of electrodes, and selects two conductors other than the one at the most distal end side, out of the at least three conductors to apply a high frequency current for cutting using the selected two conductors as a pair of electrodes.

5. The treatement instrument for endoscope according to claim 4, wherein
three conductors are provided as the conductor, and
the high-frequency current supply apparatus selects a conductor on the most distal end side and a conductor on the most proximal end side to apply a high frequency current for coagulation, and selects a central conductor and the conductor on the most proximal end side to apply a high frequency current for cutting.

6. The treatment instrument for endoscope according to claim 4, wherein
four conductors are provided as the conductor, and
the high-frequency current supply apparatus selects one or two pairs of conductors including a conductor on the most distal end side and a conductor on the most proximal end side to apply a high frequency current for coagulation, and selects two central conductors to apply a high frequency current for cutting.

7. The treatment instrument for endoscope according to any one of claims 1 to 3, wherein
the strip-shaped conductor includes at least two conductors that are disposed in parallel to each other and sandwich a non-conductor therebetween, and
the treatment instrument for endoscope comprises
a high-frequency current supply apparatus which selects at least one conductor including a conductor on the most distal end side, out of the at least two conductors to apply a high frequency current for coagulation so that the selected conductor consists one of a pair of electrodes, and selects at least one conductor other than the conductor on the most distal end side, out of the at least two conductors to apply a high frequency current for cutting so that the selected conductor consists one of a pair of electrodes.

8. The treatment instrument for endoscope according to claim 7, wherein
two conductors are provided as the conductor, and
the high-frequency current supply apparatus selects the two conductors to apply a high frequency current for coagulation and selects a conductor on the proximal end side to apply a high frequency current for cutting.

9. The treatment instrument for endoscope according to claim 7, wherein three conductors are provided as the conductor, and
the high-frequency current supply apparatus selects a conductor on the most distal end side and a conductor on the most proximal end side to apply a high frequency current for coagulation, and selects a central conductor to apply a high frequency current for cutting.

10. The treatment instrument for endoscope according to any one of claims 1 to 9, wherein
the treatment section is coupled with a distal end portion of a wire which connects between the conductor and the high-frequency source, or with a bar-shaped electrode to which the wire is connected, and
the wire is provided to be movable forward and backward relative to an insulative sheath into which the wire is inserted, and the distal end portion of the wire or the bar-shaped electrode can be adjusted to an exposed state or a non-exposed state.

11. The treatment instrument for endoscope according to any one of claims 1 to 10, wherein
discontinuous projections are integrally formed with the non-conductor, on a proximal end surface of the treatment section.

12. The treatment instrument for endoscope according to any one of claims 1 to 11, wherein
the conductor is exposed at a proximal end surface of the treatment section.

13. The treatment instrument for endoscope according to claim 1, wherein
the proximal end side non-conductor covers a circumference side surface of the conductor at a proximal end side surface of the treatment section.

14. The treatment instrument for endoscope according to claim 1, wherein
the proximal end side non-conductor covers whole of a proximal end side surface of the treatment section.

## Patentansprüche

1. Behandlungswerkzeug für ein Endoskop (10), das einen Einführabschnitt (12) zum Einführen in einen Körper aufweist, wobei der Einführabschnitt einen Behandlungsabschnitt (20, 120, 130) an einem distalen Ende des Einführabschnitts (12) besitzt,
wobei der Behandlungsabschnitt (20, 120, 130) einen Leiter (30, 124, 134) aufweist, konfiguriert zur Verbindung mit einer Hochfrequenzquelle, wobei der Behandlungsabschnitt (20, 120, 130) konfiguriert ist in einer Sandwich-Struktur, umfassend den Leiter (30, 124, 134), einen distalendseitigen Nicht-Leiter (28; 122; 132) an dem distalen Ende des Behandlungsabschnitts, und einen proximalendseitigen Nicht-Leiter (32; 126; 136) an dem anderen Ende des Leiters abgewandt von dem distalendseitigen Nicht-Leiter, wobei der Leiter sich zwischen dem distalendseitigen Nicht-Leiter und dem proximalendseitigen Nicht-Leiter befindet, wobei er in Streifenform an einer Seitenfläche des Behandlungsabschnitts freiliegt und die Seitenflächen von Leiter und Nicht-Leitern koplanar sind, und der proximalendseitige Nicht-Leiter in einer sich über den Umfang erstreckenden Streifenform an der Seitenfläche des Behandlungsabschnitts freiliegt, wobei der Behandlungsabschnitt (20, 120, 130) so ausgebildet ist, dass er eine zahnradähnliche Form aufweist, in der mehrere Spitzen (20A) und Täler (20B) abwechselnd in Umfangsrichtung des Behandlungsabschnitts (20, 120, 130) angeordnet sind, und bei Einspeisen eines Stroms in den Leiter (30, 124, 134) durch die Hochfrequenzquelle der in einer Streifenform an der Seitenfläche des Behandlungsabschnitts freiliegende Leiter (30, 124, 134) in Berührung mit einer Seite eines Teils des zu schneidenden Körpers in Berührung gebracht wird.

2. Behandlungswerkzeug für ein Endoskop nach Anspruch 1, bei dem der streifenförmige Leiter so ausgebildet ist, dass er sich um die Seitenfläche des Behandlungsabschnitts herum erstreckt.

3. Behandlungswerkzeug für ein Endoskop nach einem der Ansprüche 1 bis 2, bei dem der Behandlungsabschnitt über einen Schwenkmechanismus zum Steuern einer Lage des Behandlungsabschnitts gelagert ist.

4. Behandlungswerkzeug für ein Endoskop nach einem der Ansprüche 1 bis 3, bei dem der streifenförmige Leiter mindestens drei Leiter aufweist, die parallel zueinander mit dazwischen liegenden Nicht-Leitern angeordnet sind, und
das Behandlungswerkzeug für ein Endoskop aufweist:
eine Hochfrequenzstrom-Zuführvorrichtung, welche zwei Leiter einschließlich eines Leiters an der weitesten distal gelegenen Endseite von den mindestens drei Leitern auswählt zum Zuführen eines Hochfrequenzstroms zwecks Koagulation, wobei die ausgewählten zwei Leiter als Elektrodenpaar verwendet werden, und zwei Leiter verschieden von dem einen Leiter an der distalen Endseite von den mindestens drei Leitern auswählt zum Zuführen eines Hochfrequenzstroms zum Schneiden, wobei die ausgewählten beiden Leiter als Elektrodenpaar verwendet werden.

5. Behandlungswerkzeug für ein Endoskop nach Anspruch 4, bei dem
drei Leiter als Leiter fungieren, und
die Hochfrequenzstrom-Zuführvorrichtung eine Leiter an dem äußeren distalen Ende und eine Leiter an dem proximalen Ende auswählt zum Zuführen eines Hochfrequenzstroms zwecks Koagulation, und einen Mittelleiter und den Leiter an dem proximalen Ende auswählt zum Zuführen eines Hochfrequenzstroms zum Schneiden.

6. Behandlungswerkzeug für ein Endoskop nach Anspruch 4, bei dem
vier Leiter als Leiter vorgesehen sind, und
die Hochfrequenzstrom-Zuführvorrichtung einen von zwei Paaren von Leitern einschließlich eines Leiters am distalen Ende und eines Leiters am proximalen Ende auswählt zum Zuführen eines Hochfrequenzstroms zwecks Koagulation, und zwei Mittelleiter auswählt zum Zuführen eines Hochfrequenzstroms zum Schneiden.

7. Behandlungswerkzeug für ein Endoskop nach einem der Ansprüche 1 bis 3, bei dem
der streifenförmige Leiter mindestens zwei Leiter parallel zueinander mit dazwischenliegendem Nicht-Leiter aufweist, und
das Behandlungswerkzeug für ein Endoskop aufweist:
eine Hochfrequenzstromzuführvorrichtung, die mindestens einen Leiter einschließlich eines Leiters auf der distalen Endseite von den mindestens zwei Leitern zum Zuführen eines Hochfrequenzstroms zwecks Koagulation auswählt, so dass der ausgewählte Leiter eine Elektrode eines Elektrodenpaars bildet, und mindestens einen von dem Leiter an dem distalen Ende verschiedene Leiter von den mindestens zwei Leitern auswählt zum Zuführen eines Hochfrequenzstroms zum Schneiden, so dass der ausgewählte Leiter eine Elektrode eines Elektrodenpaars bildet.

8. Behandlungswerkzeug für ein Endoskop nach Anspruch 7, bei dem
zwei Leiter als der Leiter vorgesehen sind, und
die Hochfrequenzstrom-Zuführvorrichtung die beiden Leiter zum Zuführen eines Hochfrequenzstroms zwecks Koagulation auswählt, und einen Leiter an dem proximalen Ende zum Zuführen eines Hochfrequenzstroms zum Schneiden auswählt.

9. Behandlungswerkzeug für ein Endoskop nach Anspruch 7, bei dem
drei Leiter als der Leiter vorgesehen sind, und
die Hochfrequenzstrom-Zuführvorrichtung ein Leiter an dem distalen Ende und eine Leiter an dem proximalen Ende zum Zuführen eines Hochfrequenzstroms zwecks Koagulation auswählt, und einen Mittelleiter zum Zuführen eines Hochfrequenzstroms zum Schneiden auswählt.

10. Behandlungswerkzeug für ein Endoskop nach einem der Ansprüche 1 bis 9, bei dem
der Behandlungsabschnitt mit einem distalen Endabschnitt eines Drahts gekoppelt ist, der eine Verbindung schafft zwischen dem Leiter und der Hochfrequenzquelle oder zwischen einer stabförmigen Elektrode, an die der Draht angeschlossen ist, und
der Draht vorwärts und rückwärts in Bezug auf einen Isoliermantel bewegbar ist, in den der Draht eingeführt ist, und der distale Endabschnitt des Drahts oder die stabförmige Elektrode in einen freiliegenden Zustand und einen nicht-freiliegenden Zustand eingestellt werden kann.

11. Behandlungswerkzeug für ein Endoskop nach einem der Ansprüche 1 bis 10, bei dem diskontinuierliche Vorsprünge integriert mit dem Nicht-Leiter an einer proximalen Stirnfläche des Behandlungsabschnitts ausgebildet sind.

12. Behandlungswerkzeug für ein Endoskop nach einem der Ansprüche 1 bis 11, bei dem der Leiter an einer proximalen Stirnfläche des Behandlungsabschnitts freiliegt.

13. Behandlungswerkzeug für ein Endoskop nach Anspruch 1, bei dem der proximalendseitige Nicht-Leiter eine umfangsseitige Oberfläche des Leiters an einer proximalendseitigen Oberfläche des Behandlungsabschnitts bedeckt.

14. Behandlungswerkzeug für ein Endoskop nach Anspruch 1, bei dem der proximalendseitige Nicht-Leiter die Gesamtheit einer proximalendseitigen Fläche des Behandlungsabschnitts bedeckt.

## Revendications

1. Instrument de traitement pour un endoscope (10), comprenant une section d'introduction (12) apte à être introduite dans un corps, la section d'introduction comprenant une section de traitement (20, 120, 130) prévue sur une extrémité distale de la section d'introduction (12) ;
la section de traitement (20, 120, 130) comprend un conducteur (30, 124, 134) configuré pour être connecté à une source de haute fréquence, dans lequel la section de traitement (20, 120, 130) est configurée suivant une structure en sandwich comprenant ledit conducteur (30; 124; 134), un non-conducteur côté extrémité distale (28; 122; 132) prévu sur l'extrémité distale de la section de traitement, et un non-conducteur côté extrémité proximale (32 ; 126 ; 136) prévu sur l'autre côté du conducteur, face au non-conducteur côté extrémité distale, ledit conducteur est prévu entre le non-conducteur côté extrémité distale et le non-conducteur côté extrémité proximale de manière à être exposé suivant une forme de bande sur une surface latérale de la section de traitement, et les surfaces latérales du conducteur et des non-conducteurs sont coplanaires, et
le non-conducteur côté extrémité proximale est exposé suivant une forme de bande s'étendant de manière circonférentielle sur la surface latérale de la section de traitement, dans lequel la section de traitement (20, 120, 130) est formée de manière à présenter une forme de type engrenage, où une pluralité de crêtes (20A) et de creux (20B) sont agencés de manière alternée dans une direction circonférentielle de la section de traitement (20, 120, 130), et suite à l'application d'un courant au conducteur (30, 124, 134) par la source de haute fréquence, le conducteur (30, 124, 134), lequel est exposé suivant une forme de bande sur la surface latérale de la section de traitement, est amené en contact avec un côté d'une partie du corps à couper.

2. Instrument de traitement pour un endoscope selon la revendication 1, dans lequel
le conducteur en forme de bande est formé de manière à s'étendre autour de la surface latérale de la section de traitement.

3. Instrument de traitement pour un endoscope selon l'une quelconque des revendications 1 à 2,
dans lequel la section de traitement est supportée par un mécanisme à pivot pour commander une posture de la section de traitement.

4. Instrument de traitement pour un endoscope selon l'une quelconque des revendications 1 à 3,
dans lequel le conducteur en forme de bande inclut au moins trois conducteurs, lesquels sont disposés parallèlement les uns aux autres et prennent en sandwich entre eux les non-conducteurs, et
l'instrument de traitement pour endoscope comprend :
un appareil d'alimentation en courant de haute fréquence, lequel sélectionne deux conducteurs incluant un conducteur sur le côté d'extrémité la plus distale, parmi les au moins trois conducteurs, afin d'appliquer un courant de haute fréquence en vue d'une coagulation à l'aide des deux conducteurs sélectionnés sous la forme d'une paire d'électrodes, et sélectionne deux conducteurs autres que le un conducteur sur le côté d'extrémité la plus distale, parmi les au moins trois conducteurs, afin d'appliquer un courant de haute fréquence en vue d'une coupe à l'aide des deux conducteurs sélectionnés sous la forme d'une paire d'électrodes.

5. Instrument de traitement pour un endoscope selon la revendication 4, dans lequel trois conducteurs sont prévus pour le conducteur, et
l'appareil d'alimentation en courant de haute fréquence sélectionne un conducteur sur le côté d'extrémité la plus distale et un conducteur sur le côté d'extrémité la plus proximale, afin d'appliquer un courant de haute fréquence en vue d'une coagulation, et sélectionne un conducteur central et le conducteur sur le côté d'extrémité la plus proximale afin d'appliquer un courant de haute fréquence en vue d'une coupe.

6. Instrument de traitement pour un endoscope selon la revendication 4, dans lequel quatre conducteurs sont prévus pour le conducteur, et
l'appareil d'alimentation en courant de haute fréquence sélectionne une ou deux paires de conducteurs incluant un conducteur sur le côté d'extrémité la plus distale et un conducteur sur le côté d'extrémité la plus proximale, afin d'appliquer un courant de haute fréquence en vue d'une coagulation, et sélectionne deux conducteurs centraux afin d'appliquer un courant de haute fréquence en vue d'une coupe.

7. Instrument de traitement pour un endoscope selon l'une quelconque des revendications 1 à 3, dans lequel
le conducteur en forme de bande inclut au moins deux conducteurs, lesquels sont disposés parallèlement les uns aux autres et prennent en sandwich entre eux un non-conducteur, et
l'instrument de traitement pour endoscope comprend :
un appareil d'alimentation en courant de haute fréquence, lequel sélectionne au moins un conducteur incluant un conducteur sur le côté d'extrémité la plus distale, parmi les au moins deux conducteurs, afin d'appliquer un courant de haute fréquence en vue d'une coagulation, de sorte que le conducteur sélectionné consiste en une électrode d'une paire d'électrodes, et sélectionne au moins un conducteur autre que le conducteur sur le côté d'extrémité la plus distale, parmi les au moins deux conducteurs, afin d'appliquer un courant de haute fréquence en vue d'une coupe, de sorte que le conducteur sélectionné consiste en une électrode d'une paire d'électrodes.

8. Instrument de traitement pour un endoscope selon la revendication 7, dans lequel deux conducteurs sont prévus pour le conducteur, et
l'appareil d'alimentation en courant de haute fréquence sélectionne les deux conducteurs afin d'appliquer un courant de haute fréquence en vue d'une coagulation, et sélectionne un conducteur sur le côté d'extrémité proximale afin d'appliquer un courant de haute fréquence en vue d'une coupe.

9. Instrument de traitement pour un endoscope selon la revendication 7, dans lequel trois conducteurs sont prévus pour le conducteur, et
l'appareil d'alimentation en courant de haute fréquence sélectionne un conducteur sur le côté d'extrémité la plus distale et un conducteur sur le côté d'extrémité la plus proximale afin d'appliquer un courant de haute fréquence en vue d'une coagulation, et sélectionne un conducteur central afin d'appliquer un courant de haute fréquence en vue d'une coupe.

10. Instrument de traitement pour un endoscope selon l'une quelconque des revendications 1 à 9, dans lequel
la section de traitement est couplée avec une portion d'extrémité distale d'un fil, lequel est connecté entre le conducteur et la source de haute fréquence, ou avec une électrode en forme de barre, à laquelle le fil est connecté, et
le fil est prévu de manière à pouvoir se déplacer vers l'avant et vers l'arrière par rapport à une gaine isolante dans laquelle le fil est introduit, et la portion d'extrémité distale du fil ou de l'électrode en forme de barre peut être ajustée sur un état exposé ou un état non exposé.

11. Instrument de traitement pour un endoscope selon l'une quelconque des revendications 1 à 10,
dans lequel des saillies discontinues sont formées d'un seul tenant avec le non-conducteur, sur une surface d'extrémité proximale de la section de traitement.

12. Instrument de traitement pour un endoscope selon l'une quelconque des revendications 1 à 11,
dans lequel le conducteur est exposé sur une surface d'extrémité proximale de la section de traitement.

13. Instrument de traitement pour un endoscope selon la revendication 1,
dans lequel le non-conducteur côté extrémité proximale couvre une surface latérale de circonférence du conducteur sur une surface côté extrémité proximale de la section de traitement.

14. Instrument de traitement pour un endoscope selon la revendication 1,
dans lequel le non-conducteur côté extrémité proximale couvre la totalité d'une surface côté extrémité proximale de la section de traitement.
